# DEMANDE DE BREVET EUROPEEN

(11) **EP 4 782 039 A1**
(43) Date de publication de la demande: **29.07.2026**
(21) Numéro de dépôt: 26152159.5
(22) Date de dépôt: 15.01.2026
(51) Int. Cl.: A61M 16/00, A61M 16/20

(54) **APPAREIL DE THÉRAPIE RESPIRATOIRE DÉLIVRANT UNE ASSISTANCE RESPIRATOIRE INTERMITTENTE PAR PRESSION POSITIVE AVEC PLATEAU DE PRESSION**

(30) Priorité: 23.01.2025 FR 2500692
(71) Demandeur: EOVE, 64000 Pau (FR)
(72) Inventeur: JOURDAIN, Cedric, 64000 Pau (FR); LIU, Tingting, 64000 Pau (FR); GALBRUN, Marie-Amedee, 64000 Pau (FR); TEILLET, Manuel, 64000 Pau (FR)
(74) Mandataire: Air Liquide

(57) **Abrégé**

L'invention concerne un appareil de thérapie respiratoire (1) pour fournir une assistance respiratoire intermittente par pression positive à une personne, comprenant: une turbine motorisée (2); un capteur de débit (5) agencé sur un circuit de gaz interne (3); un dispositif anti-retour (6) agencé dans le circuit de gaz interne, en aval du capteur de débit; un capteur de pression proximale (7) agencé sur un circuit patient externe (10); et des moyens de pilotage (4) configurés pour, lors de chaque phase inspiratoire,
- commander la turbine pour fournir un débit gazeux régulé, pendant une durée d'insufflation de gaz non-nulle suffisante pour atteindre un seuil de pression maximale fixé,
- et lorsqu'une pression mesurée est supérieure ou égale au seuil de pression maximale préfixé, commander la turbine pour fournir une pression de plateau correspondant au seuil de pression maximale.

## Description

L'invention concerne un appareil de thérapie respiratoire délivrant une assistance respiratoire intermittente par pression positive ou IPPB (pour *Intermittent Positive Pressure Breathing* en anglais), aussi appelé « appareil IPPB », avec mise en œuvre d'un plateau de pression en fin d'inspiration, c'est-à-dire un maintien d'une pression constante pendant une durée de plateau préfixée (non nulle).

D'une façon générale, fournir une thérapie respiratoire de type IPPB (appelée « thérapie IPPB » ci-après) à certaines personnes, aussi appelés « patients », souffrant de problèmes ou pathologies respiratoires permet notamment d'améliorer :
- le drainage bronchique en délivrant un volume inspiratoire important permettant d'augmenter l'efficacité du débit expiratoire et de la toux ;
- la fonction respiratoire en allant au-delà de l'inspiration maximale du patient et en augmentant ainsi sa capacité vitale ; et
- le recrutement pulmonaire, en particulier des zones pulmonaires mal ou non ventilées.

Par exemple, EP4079356 enseigne un appareil d'assistance à la toux permettant d'opérer des insufflations de gaz aux patients souffrant de troubles respiratoires nécessitant une aide pour évacuer de leurs sécrétions pulmonaires et/ou pour améliorer leur élasticité pulmonaire et thoracique, que ces patients soient des patients adultes ou pédiatriques, lequel peut être utilisé facilement par une personne n'ayant pas ou peu de connaissances médicales, typiquement une auxiliaire de vie, une personne de l'entourage familial du patient, encore appelés « les aidants ».

D'autres appareils de thérapie respiratoire sont décrits par DE102014001218, WO2015/110098, EP1502619 et US2013/047983.

D'une façon générale, la thérapie IPPB repose sur l'application d'un débit gazeux constant en inspiration jusqu'à atteindre une pression maximale donnée, suivi d'une expiration avec ou sans pression expiratoire positive (PEP). La synchronisation avec les phases respiratoires du patient se fait grâce à une détection de l'effort du patient, typiquement l'appel de gaz en début d'inspiration, ou par un mécanisme ou dispositif de déclenchement manuel de l'inspiration, par exemple via un appui sur un bouton ou une touche de commande.

Il existe plusieurs types de dispositifs ou d'appareils de thérapie respiratoire permettant de mettre en œuvre une thérapie IPPB mais ceux-ci présentent tous des inconvénients plus ou moins importants.

Actuellement, les dispositifs de thérapie IPPB fonctionnent sur la base d'une régulation du débit d'inspiration, c'est-à-dire du débit de gaz (e.g. air) fourni aux voies aériennes du patient pendant son traitement.

Le débit fourni par la source de gaz, telle une turbine ou autre, du dispositif ou appareil de fourniture de gaz, est habituellement constant pendant chaque phase inspiratoire du patient, ce qui se traduit par une courbe de débit de forme rectangle. Maintenir le débit fourni constant permet un remplissage progressif des poumons du patient et une augmentation progressive simultanée de la pression gazeuse dans les voies aériennes, en particulier dans les poumons du patient.

La/chaque phase inspiratoire du patient se termine lorsqu'une pression maximale préfixée est atteinte, i.e. un seuil de pression maximale réglable. Autrement dit, pour terminer l'inspiration (i.e. une phase inspiratoire), on utilise le seuil de pression qui a été réglé par l'utilisateur, typiquement un personnel soignant, qui permet de déclencher un passage en expiration (i.e. phase expiratoire) lorsqu'il est atteint. Procéder ainsi vise à mieux contrôler la pression maximale de coupure pour maximiser le confort du patient et limiter les risques de barotraumatisme.

D'une façon générale, l'obtention d'un volume maximal dans les poumons du patient est le but de la séance de traitement et l'augmentation de la pression de coupure est un moyen d'y parvenir.

Cependant, il a été constaté en pratique que l'efficacité du traitement et/ou le confort des patients, ainsi que l'observance au traitement, dépendaient notamment des physiologies des patients eux-mêmes, notamment en termes de compliance et résistance, mais aussi du type d'interface respiratoire utilisée pour administrer le gaz, i.e. masque respiratoire avec ou sans fuites.

Un problème est dès lors de proposer un appareil ou dispositif de thérapie respiratoire permettant de délivrer une assistance respiratoire intermittente par pression positive, c'est-à-dire une thérapie de type IPPB, ou « appareil IPPB », qui soit amélioré, notamment qui permette de palier tout ou partie des inconvénients susmentionnés, en particulier de délivrer une thérapie IPPB plus efficace avec un confort accru pour le patient et/ou une meilleure observance et ce, préférentiellement quelle que soit la physiologie du patient considéré (e.g. compliance et résistance), et/ou prendre mieux en compte les caractéristiques du type d'interface respiratoire utilisée et/ou des accessoires susceptibles d'être mis en œuvre durant le traitement, tels que nébuliseur, filtre....

Une solution selon l'invention concerne un appareil (i.e. dispositif ou installation) de thérapie respiratoire, c'est-à-dire un « appareil IPPB », pour fournir une assistance respiratoire intermittente par pression positive (IPPB) à une personne, i.e. un patient, comprenant :
- une coque ou carcasse périphérique,
- une turbine motorisée pour fournir un gaz respiratoire sous pression,
- un circuit de gaz interne pour convoyer le gaz respiratoire délivré par la turbine,
- un capteur de débit agencé sur le circuit de gaz interne,
- un dispositif anti-retour agencé dans le circuit de gaz interne, en aval du capteur de débit,
- un circuit patient externe venant se raccorder fluidiquement au circuit de gaz interne pour convoyer le gaz respiratoire provenant du circuit de gaz interne,
- un capteur de pression proximale agencé pour permettre une mesure de la pression (i.e. des mesures de pression instantanée) au sein du circuit patient externe à proximité de la valve expiratoire, et
- des moyens de pilotage raccordés au capteur de pression proximale et à la turbine motorisée, et configurés in la turbine en fonction d'au moins une mesure de pression opérée par le capteur de pression proximale et d'au moins une mesure de débit opérée par le capteur de débit.

La turbine, les moyens de pilotage, le circuit interne, le capteur de débit et le dispositif anti-retour sont agencés dans la coque ou carcasse périphérique de l'appareil, c'est-à-dire à l'intérieur de l'appareil.

De plus, dans l'appareil IPPB, les moyens de pilotage sont configurés pour, lors de chaque phase inspiratoire de la personne, i.e. du patient :
a) commander la turbine pour fournir un débit gazeux régulé (Q_{réglé}), pendant une durée d'insufflation de gaz (Di) non-nulle suffisante pour atteindre un seuil de pression maximale (Pₘₐₓ) fixé,
b) et lorsqu'une pression mesurée (Pₘₑₛ) par le capteur de pression proximale est (i.e., est ou devient) supérieure ou égale au seuil de pression maximale (Pₘₐₓ) préfixé (i.e. Pₘₑₛ = Pₘₐₓ), commander la turbine pour fournir une pression de plateau (Pₚₗₐₜ) correspondant au seuil de pression maximale (Pₘₐₓ) de manière à maintenir au moins une partie du circuit patient externe à ladite pression de plateau (Pₚₗₐₜ), pendant une durée de plateau (Dp) non-nulle donnée.

Autrement dit, selon l'invention, pendant la durée d'insufflation de gaz (Di), la turbine (i.e. son moteur) est pilotée en débit, alors qu'ensuite, pendant la durée de plateau (Dp), elle est pilotée en pression.

Grâce au maintien du circuit patient externe à la pression de plateau (Pₚₗₐ) qui est égale au seuil de pression maximale (Pₘₐₓ) préfixé, pendant la durée de plateau (Dp), on peut augmenter le confort pour le patient et/ou l'efficacité du traitement global et/ou améliorer l'observance du patient pour son traitement.

En effet, la phase de plateau de pression permet d'augmenter le volume de gaz, i.e. d'air, délivré au patient, i.e. à la personne soignée, et d'améliorer la propagation du gaz dans les poumons sans pour autant augmenter la pression maximale de coupure, c'est-à-dire la pression maximale délivrée. Le fait de ne pas augmenter la pression de coupure est une façon de limiter les éventuels effets secondaires du traitement de type barotrauma sur les poumons.

Autrement dit, grâce à la phase de plateau de pression, un personnel soignant, i.e. un clinicien ou analogue, peut adapter la thérapie du patient pour obtenir le meilleur compromis possible entre la pression maximale et l'efficacité du traitement, ce qui est dans l'intérêt du patient ainsi traité.

Selon le mode de réalisation considéré, l'appareil de l'invention, c'est-à-dire « l'appareil IPPB », peut comprendre l'une ou plusieurs des caractéristiques suivantes :
- les moyens de pilotage sont configurés pour commander le moteur électrique de la turbine pour fournir un débit gazeux et/ou la pression désirés.
- le moteur électrique de la turbine est préférentiellement sans balai et/ou à courant continu.
- les moyens de pilotage sont configurés pour commander la turbine, i.e. son moteur, pour que le débit gazeux régulé (Q_{réglé}) fourni à l'étape a), soit un débit (approximativement) constant ou décroissant pendant la durée d'insufflation de gaz (Di). Bien entendu, le débit peut présenter de légères variations ou fluctuations autour de la valeur de débit désirée, par exemple de l'ordre de +/-5%.
- il comprend des moyens de réglage de durée configurés pour permettre à l'utilisateur de régler la durée de plateau (Dp).
- de préférence, la durée de plateau (Dp) est comprise entre 0,1 et 10 sec , de préférence entre 0,5 et 5 sec.
- il comprend des moyens de réglage de pression configurés pour permettre à l'utilisateur de régler le seuil de pression maximale (Pₘₐₓ).
- de préférence, le seuil de pression maximale (Pₘₐₓ) est compris entre 10 et 50 cmH2O.
- il comprend des moyens de réglage de débit pour permettre à l'utilisateur de régler la valeur de débit (Q_{réglé}) désirée.
- de préférence, la valeur de débit (Q_{réglé}) est comprise entre 1 et 200 L/min, de préférence entre 1 et 150 L/min, préférentiellement encore entre 5 et 100 L/min.
- le débit gazeux (Q_{réglé}) fourni pendant la durée d'insufflation de gaz (Di) est de préférence constant ou approximativement constant.
- les moyens de réglage de durée et/ou les moyens de réglage de pression et/ou de réglage de débit comprennent un ou des boutons, touches ou analogues, en particulier un ou des boutons affichés sur un écran tactile.
- les moyens de pilotage sont configurés pour comparer une valeur de pression proximale mesurée (Pₘₑₛ) par le capteur de pression proximale au seuil de pression maximale (Pₘₐₓ) fixé et déclencher l'étape b) lorsque la valeur de pression mesurée (Pₘₑₛ) par le capteur de pression proximale (7) est ou dévient égale au seuil de pression maximale (Pmax) préfixé, i.e. Pₘₑₛ = Pₘₐₓ.
- le maintien d'au moins une partie du circuit patient externe à la pression de plateau (Pₚₗₐ), pendant une durée de plateau (Dp) non-nulle donnée, se fait via la régulation de pression de l'étape b).
- une valve expiratoire est agencée sur le circuit patient externe.
- à la fin de la durée de plateau (Dp), la valve expiratoire s'ouvre pour évacuer vers l'atmosphère au moins une partie de la pression gazeuse présente dans le circuit patient externe.
- la valve expiratoire s'ouvre en réponse à une baisse de pression dans le circuit de gaz interne à la fin de la durée de plateau (Dp).
- une ligne de pilotage pneumatique de la valve expiratoire vient se raccorder fluidiquement au circuit de gaz interne entre la turbine et le capteur de débit.
- la ligne de pilotage pneumatique coopère avec la valve expiratoire pour contrôler l'ouverture ou la fermeture de ladite valve expiratoire.
- l'ouverture de la valve expiratoire permet une évacuation de gaz, i.e. de pression gazeuse, du circuit patient externe vers l'atmosphère ambiante et une diminution de pression dans tout ou partie du circuit patient externe.
- une ligne de pilotage pneumatique est une liaison pneumatique, typiquement un tuyau flexible, de préférence en polymère.
- les moyens de pilotage sont raccordés au capteur de pression proximale, c'est-à-dire que le capteur de pression peut être, selon le mode de réalisation considéré, relié électriquement auxdits moyens de pilotage et/ou être (directement) intégrés auxdits moyens de pilotage, de manière à coopérer avec lesdits moyens de pilotage.
- de préférence, le capteur de pression proximale est intégré aux moyens de pilotage, en particulier porté par une carte électronique faisant partie des moyens de pilotage.
- le capteur de pression proximale comprend une ligne de mesure de pression venant se raccorder au circuit patient en aval de la valve expiratoire.
- le capteur de pression proximale est configuré pour permettre une mesure de la pression au sein du circuit patient externe entre la valve expiratoire et une interface respiratoire assurant les échanges gazeux avec le patient, typiquement lors des phases inspiratoires, et/ou éventuellement lors des phases expiratoires dudit patient.
- le circuit de gaz interne comprend un passage, un conduit ou analogue servant à véhiculer du gaz.
- l'interface respiratoire est un masque respiratoire, une sonde de trachéostomie ou un embout buccal.
- le circuit de gaz interne comprend une sortie de gaz.
- le circuit patient externe vient se raccorder fluidiquement à ladite sortie de gaz du circuit de gaz interne.
- le circuit patient externe comprend un tuyau flexible, typiquement en polymère.
- le circuit patient externe peut aussi comprendre ou mettre en œuvre des éléments de connexion fluidique additionnels de type connecteurs, raccords ou analogues.
- le circuit patient externe comprend une branche unique ou simple branche
- selon un autre mode de réalisation, le circuit patient externe peut être à double branche.
- les moyens de pilotage comprennent au moins un microprocesseur.
- ledit au moins un microprocesseur est agencé sur au moins une carte électronique.
- les moyens de pilotage comprennent au moins une carte électronique.
- la turbine motorisée comprend un moteur électrique.
- le moteur électrique est alimenté électriquement (110/220V).
- les moyens de pilotage sont configurés pour commander les accélérations du moteur électrique de la turbine, et/ou les décélérations ou freinages dudit moteur. Par décélération, on entend un freinage ou ralentissement du moteur, par exemple opéré par inversion des phases du moteur.
- le dispositif anti-retour comprend un clapet anti-retour, une valve anti-retour ou analogue.
- le dispositif anti-retour est configuré pour s'opposer à tout flux négatif, c'est-à-dire remontant en direction de la turbine, i.e. s'écoulant dans le sens opposé au sens normal de circulation du gaz de la turbine vers le patient.
- la valve expiratoire est préférentiellement une valve à au moins 2 voies.
- selon un autre mode de réalisation, la valve expiratoire peut être une valve de PEP.
- le moteur électrique de la turbine est piloté, i.e. commandé ou contrôlé, par les moyens de pilotage.
- la ligne de pilotage pneumatique comprend un tuyau flexible en polymère de petit diamètre, typiquement d'environ 2 à 10 mm de diamètre.
- il comprend une coque ou carcasse périphérique formée d'une ou plusieurs sous-parties fixées entre elles, par exemple par vissage.
- la coque ou carcasse périphérique peut être en polymère rigide.
- il comprend une interface graphique utilisateur ou IGU.
- l'IGU peut comprend un écran d'affichage, de préférence de type tactile, et/ou des touches de sélection ou de validation, notamment des touches tactiles affichées sur l'écran.
- il comprend des moyens de mémorisation, telle une mémoire RAM ou EEPROM, ou une mémoire flash, ou autre.
- les moyens de pilotage commandent un début de ralentissement ou un freinage de la turbine lorsque lesdits moyens de pilotage déterminent à partir de la la pression proximale mesurée, typiquement au plus près du patient, la fin de la phase d'insufflation de gaz (Di).
- les moyens de pilotage sont configurés pour comparer la valeur de pression proximale mesurée par le capteur de pression proximale à la valeur de pression maximale (pré)réglée, c'est-à-dire le seuil de pression maximale (Pₘₐₓ), et en déduire la fin de la durée d'insufflation de gaz (Di) et/ou le début de la durée de plateau (Dp).
- les moyens de pilotage sont configurés pour déclencher l'étape b) en réponse à la détection d'une pression proximale (i.e. une pression instantanée) égale au seuil de pression maximale (Pₘₐₓ).
- le moteur de la turbine est configuré pour pouvoir tourner à une vitesse de rotation pouvant atteindre 50 000 tours/min, voire 70 000 tr/min.
- les moyens de pilotage comprennent un compteur temporel permettant de déterminer ou mesurer les durées.
- le seuil de pression maximale (Pₘₐₓ) est mémorisé par les moyens de mémorisation.
- le seuil de pression maximale (Pₘₐₓ) est (pré)fixé, i.e. réglé par l'utilisateur (i.e. personnel médical), de préférence via l'IGU.
- la durée de plateau (Dp) non-nulle donnée est mémorisée par les moyens de mémorisation.
- la durée de plateau (Dp) non-nulle donnée est (pré)fixée, i.e. réglée par l'utilisateur (i.e. personnel médical), de préférence via l'IGU.
- la valve d'expiration est configurée pour s'ouvrir automatiquement dès le début de la décélération (i.e. freinage) de la turbine, i.e. c'est-à-dire lorsque (le moteur de) la turbine ralentit, typiquement après la phase de plateau de pression. Ceci est possible grâce au raccordement fluidique de la ligne de pilotage de la valve d'expiration en aval de la turbine et en amont du dispositif anti-retour, c'est-à-dire entre turbine et dispositif anti-retour de gaz, et au pilotage pneumatique (i.e. en pression) de ladite valve d'expiration par ladite ligne de pilotage.
- la valve d'expiration comprend un corps de valve comprenant des moyens d'étanchéité flexibles.
- les moyens d'étanchéité flexibles comprennent une membrane, un ballonnet ou analogue, de préférence flexible et/ou déformable, typiquement une membrane.
- la ligne de pilotage pneumatique contrôle l'ouverture et/ou la fermeture de la valve d'expiration en agissant pneumatiquement, c'est-à-dire via une fourniture de gaz sous pression, sur les moyens d'étanchéité flexibles du corps de valve.
- le corps de valve de la valve d'expiration comprend au moins un orifice de mise à l'atmosphère.
- les moyens d'étanchéité flexibles sont configurés pour autoriser ou, à l'inverse, pour empêcher une circulation du flux gazeux au travers de l'orifice de mise à l'atmosphère, c'est-à-dire une décharge de gaz à l'atmosphère.
- les moyens d'étanchéité flexibles sont agencés sur un passage de flux gazeux traversant le corps de valve.
- les moyens d'étanchéité flexibles sont configurés pour coopérer avec au moins une partie de la paroi du corps de valve pour contrôler ou assurer l'étanchéité gazeuse, en particulier au moins une partie de la paroi du passage de flux gazeux agencé dans le corps de valve.
- le circuit de gaz interne comprend une sortie de gaz, par exemple portée par un raccord ou embout de sortie ou analogue.
- le circuit patient externe vient se raccorder fluidiquement à la sortie de gaz du circuit de gaz interne, par exemple vient se connecter au raccord ou embout de sortie ou analogue du circuit de gaz interne.
- durant la durée de plateau Dp, le débit délivré par la turbine diminue progressivement.
- un débit gazeux résiduel est maintenu pendant au moins une partie de la durée de plateau Dp.
- pendant la durée de plateau Dp, la valve expiratoire est fermée de manière à maintenir d'au moins une partie du circuit patient externe à la pression de plateau Pₚₗₐ.
- à la fin de la durée de plateau Dp, la valve expiratoire s'ouvre de manière à évacuer au moins une partie de la pression gazeuse présente dans le circuit patient externe.
- la valve expiratoire est maintenue fermée pendant les durées d'inspiration Di et de plateau Dp.

Par ailleurs, l'invention concerne aussi une méthode pour fournir une thérapie respiratoire de type IPPB à une personne en ayant besoin, i.e. un patient, dans laquelle on utilise un appareil (i.e. dispositif ou installation) de thérapie respiratoire, comme décrit ci-avant ou ci-après, pour fournir un gaz respiratoire à ladite personne, dans laquelle, lors de chaque phase inspiratoire de la personne :
i. on fournit un débit gazeux régulé (Q_{réglé}), pendant une durée d'insufflation de gaz (Di) non-nulle suffisante pour atteindre un seuil de pression maximale (Pₘₐₓ) fixé, et
ii. lorsqu'une pression mesurée (Pₘₑₛ) par le capteur de pression proximale est supérieure ou égale au seuil de pression maximale (Pₘₐₓ) préfixé, on fournit une pression de plateau (Pₚₗₐₜ) correspondant au seuil de pression maximale (Pₘₐₓ) de manière à maintenir au moins une partie du circuit patient externe à ladite pression de plateau (Pₚₗₐₜ), pendant une durée de plateau (Dp) non-nulle donnée.

Aux étapes i) et ii), le débit gazeux et la pression gazeuse sont fournies par la turbine de l'appareil qui est pilotée par les moyens de pilotage de l'appareil.

Dans le cadre de la présente invention:
- on appelle « capteur de pression », tout dispositif ou moyen permettant de déterminer une pression de gaz.
- on appelle « capteur de débit », tout dispositif ou moyen permettant de déterminer un débit de gaz, y compris indirectement via des mesures de pression.
- les termes « appareil » et « dispositif » sont considérés comme équivalents et substituables.
- les termes « turbine », « compresseur », « soufflante » ou analogue sont considérés comme équivalents et substituables.
- les termes « valve » et « vanne » sont considérés comme équivalents et substituables.
- les termes « moyens » et « dispositif » sont considérés comme équivalents et substituables, par exemple les termes « moyens de pilotage » sont considérés comme équivalents et substituables par les termes « dispositif de pilotage ».
- les termes « piloté », « commandé » et « contrôlé » sont considérés comme équivalents et substituables.
- les termes « piloter », « commander » et « contrôler » sont considérés comme équivalents et substituables
- on considère que la respiration d'une personne comprend ou est formée de cycles respiratoires successifs, chaque cycle respiratoire comprenant une phase inspiratoire suivie d'une phase expiratoire.
- pendant une phase inspiratoire, une personne inhale/inspire dans ses poumons du gaz respiratoire, tel de l'air ou un mélange air/O₂.
- pendant une phase expiratoire, une personne exhale/expire de ses poumons du gaz contenant du CO₂.

L'invention va maintenant être mieux comprise grâce à la description détaillée suivante, faite à titre illustratif mais non limitatif, en référence aux figures annexées parmi lesquelles :
Fig. 1 est un schéma de principe d'un mode de réalisation d'un appareil de thérapie respiratoire selon l'invention.
Fig. 2 schématise (vue extérieure partielle) un mode de réalisation d'un appareil selon l'invention, tel celui de Fig. 1, avec son raccordement au secteur.
Fig. 3 schématise des courbes de débit (Q) et pression (P) obtenues avec un appareil IPPB selon l'invention.

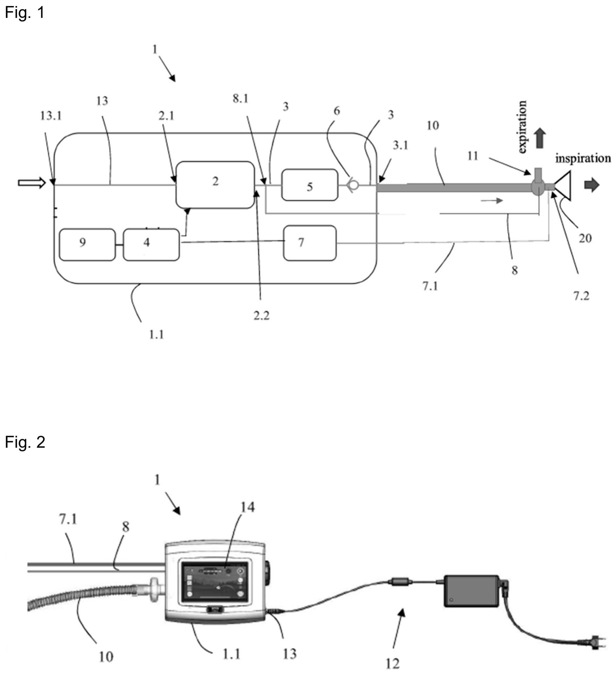

Fig. 1 et Fig. 2 schématisent un mode de réalisation d'un appareil ou dispositif 1 de thérapie respiratoire selon l'invention permettant de fournir une assistance respiratoire intermittente par pression positive, appelée « thérapie IPPB » ci-après, à une personne en ayant besoin, à savoir typiquement un patient à traiter ou analogue.

Comme illustré sur le schéma de principe de Fig. 1, l'appareil de thérapie respiratoire 1 ou « appareil IPPB » comprend une turbine 2 motorisée (aussi appelée (micro-)soufflante, pompe, compresseur ou analogue) combinée à un système de commande passif comprenant d'une valve d'expiration 11, aussi appelée valve expiratoire, et à des moyens de mesure de pression proximale, typiquement un capteur de pression proximale 7, comme décrit ci-après, dans le but de délivrer la thérapie IPPB au patient de manière efficace et avec un confort accru pour le patient, notamment en termes de limitation ou de réduction des nuisances sonores, c'est-à-dire du bruit généré lors du fonctionnement de l'appareil 1.

Plus précisément, la turbine 2 motorisée comprend un moteur électrique, typiquement sans balai à courant continu, qui est piloté, i.e. contrôlé, par des moyens de pilotage 4, typiquement par un (des) microprocesseur agencé sur une (des) carte électronique. La turbine 2 est alimentée en air ambiant par une ligne d'amenée d'air 13, tel un conduit, un passage ou analogue, qui relie une entrée d'air 13.1 à l'entrée 2.1 de la turbine 2.

La turbine 2 motorisée a une architecture classique, à savoir qu'elle comprend schématiquement un moteur électrique agencé dans un carter de protection, lequel est surmonté d'une volute comprenant un compartiment interne dans lequel est agencée une roue à ailettes. La roue à ailettes est portée par l'axe du moteur. Elle est entrainée en rotation par l'axe du moteur, lors de son fonctionnement, de manière à d'aspirer le gaz respiratoire, tel de l'air, qui pénètre alors dans le compartiment interne de la volute, puis en ressort avant d'être envoyé vers le patient.

La turbine 2 fournit ensuite le gaz respiratoire sous pression, tel de l'air, à un circuit de gaz 3 interne servant à acheminer, i.e. convoyer, le gaz respiratoire délivré en sortie 2.2 de turbine 2 jusqu'à une interface respiratoire 20, tel un masque respiratoire, un embout buccal ou une sonde de trachéostomie, fournissant le gaz respiratoire aux voies aériennes du patient à traiter.

Lorsque le moteur électrique de la turbine 2 (i.e. sa vitesse de rotation) est contrôlé par les moyens de pilotage 4, son axe portant la roue à ailettes est entrainé en rotation, ce qui permet de fournir le gaz au débit souhaité, par exemple au débit régulé (Q_{réglé}), qui peut être constant ou variable, selon le contrôle opéré, comme expliqué ci-après.

A l'inverse, les moyens de pilotage 4 peuvent aussi contrôler la vitesse de rotation du moteur pour le freiner ou le ralentir. Dans ce cas, la vitesse de rotation de l'axe-moteur portant la roue à ailettes diminue plus ou moins rapidement et la fourniture de gaz diminue alors en corrélation avec le ralentissement/freinage du moteur.

Un exemple de turbine ou (micro-)soufflante pour appareil médical de ventilation est donné par EP2947328 mais, bien entendu, d'autres types de turbines peuvent convenir.

Utiliser une turbine motorisée 2 pour délivrer le gaz permet d'obtenir une limitation du bruit généré pendant le fonctionnement de l'appareil 1.

Afin de convoyer le gaz respiratoire provenant du circuit de gaz 3 interne, c'est-à-dire agencé dans la coque 1.1 de l'appareil 1, jusqu'à l'interface respiratoire 20, on prévoit un circuit patient 10 externe, typiquement un (des) conduit ou tuyau flexible, qui vient se raccorder fluidiquement à une sortie de gaz 3.1 du circuit de gaz 3 interne, typiquement un tuyau flexible en polymère. La sortie de gaz 3.1 peut être portée par un connecteur ou raccord de sortie agencé sur la carcasse 1.1 de l'appareil 1. Le circuit patient 10 externe vient se raccorder fluidiquement audit raccord de sortie, par exemple s'y emboiter, s'y visser, s'y fixer via un système à baïonnette ou tout autre système de raccordement adapté, de préférence le tuyau flexible comprend des moyens de connexion fluidique complémentaires du raccord de sortie. Le circuit patient 10 se situe donc à l'extérieur de la coque 1.1 de l'appareil 1.

Afin de mesurer le débit de gaz au sein de l'appareil 1, un capteur de débit 5 est agencé sur le circuit de gaz 3 interne, c'est-à-dire en aval de la sortie de la turbine 2 et en amont de la sortie de gaz 3.1 du circuit de gaz 3. Le capteur de débit 5 fournit les mesures de débit aux moyens de pilotage 4, où ils sont traités afin de réguler le fonctionnement de la turbine 2, typiquement les accélérations ou le freinage de son moteur électrique. Le capteur de débit 5 est raccordé électriquement aux moyens de pilotage 4, typiquement par des liaisons électriques, tels des câbles électriques ou analogues, afin de lui transmettre les mesures de débit réalisées.

On peut aussi utiliser ces mesures de débit pour vérifier le bon fonctionnement du dispositif anti-retour 6, e.g. clapet anti-retour, en s'assurant qu'il n'existe aucun flux négatif remontant vers la turbine 2, lors des phases expiratoires du patient, c'est-à-dire de vérifier que le dispositif anti-retour 6 garantit une étanchéité fluidique dans le sens négatif.

Le circuit patient 10 externe comprend, quant à lui, la valve expiratoire 11 servant à contrôler les rejets de gaz expirés par le patient et/ou tout ou partie de la pression gazeuse présente dans le circuit patient 10, en particulier après la phase de plateau comme expliqué ci-après, vers l'atmosphère ambiante étant donné que cette valve expiratoire 11 permet de mettre en relation la partie aval du circuit patient 10 avec l'extérieur, c'est-à-dire l'atmosphère ambiante, en particulier en fin de phase inspiratoire et/ou lors des phases expiratoires du patient.

Par ailleurs, afin d'empêcher les remontées de gaz/pression et permettre leur meilleure évacuation via la valve expiratoire 11, un dispositif anti-retour 6, c'est-à-dire un clapet anti-retour, une valve unidirectionnelle ou analogue, est agencé sur le circuit de gaz 3 interne, en aval du capteur de débit 5 et en amont de la sortie de gaz 3.1 du circuit de gaz 3. Un tel dispositif anti-retour 6 présente en outre l'avantage d'être simple à mettre en œuvre et de ne demander aucun pilotage particulier, donc d'être aussi de coût bas.

La présence de ce dispositif anti-retour 6, typiquement un clapet anti-retour, permet aussi d'améliorer le fonctionnement de l'appareil 1, typiquement l'ouverture de la valve expiratoire 11 lors des phases expiratoires, comme expliqué ci-après.

La valve expiratoire 11, en particulier son ouverture et/ou sa fermeture, est commandée ou contrôlée par une ligne de pilotage pneumatique 8, tel un conduit flexible, venant se raccorder fluidiquement 8.1 au circuit de gaz 3 interne entre la turbine 2 et le capteur de débit 5. La ligne de pilotage pneumatique 8 coopère avec la valve expiratoire 11 en lui fournissant une pression gazeuse servant à contrôler l'ouverture ou la fermeture de ladite valve expiratoire 11, c'est-à-dire son degré d'ouverture/fermeture.

Préférentiellement, l'ouverture et/ou la fermeture de ladite valve expiratoire 11 est/sont contrôlée(s) par des moyens d'étanchéité flexibles sur lesquels agit la pression gazeuse apportée par la ligne de pilotage pneumatique 8, comme expliqué ci-après.

Enfin, le circuit patient 10 externe comprend aussi un capteur de pression proximale 7 agencé de manière à permettre une mesure de la pression, i.e. de la pression instantanée, au sein du circuit patient 10 et à proximité 7.2 de la valve expiratoire 11, typiquement entre la valve expiratoire 11 et l'interface respiratoire 20.

Ce capteur de pression proximale 7 permet de détecter les appels de gaz du patient, c'est-à-dire ses inspirations, mais aussi si le seuil de pression maximale (Pₘₐₓ) fixé a été atteint en fin de phase inspiratoire, comme détaillé ci-après.

Le capteur de pression proximale 7 est raccordé aux moyens de pilotage 4, c'est-à-dire qu'il coopère avec ceux-ci, de sorte que ces derniers puissent piloter la turbine 2 à partir de tout ou partie des mesures opérées par le capteur de pression proximale 7. De préférence, le capteur de pression proximale 7 est directement intégré aux moyens de pilotage 4, par exemple porté par une carte électronique desdits moyens de pilotage 4.

L'utilisation d'une turbine motorisée 2 pilotée assure un confort accru tant au niveau du plus faible bruit généré par l'appareil 1 lors d'un traitement IPPB, que de l'adaptabilité du profil de débit délivré au patient, par exemple un débit en « rectangle » ou autre.

Par ailleurs, la mesure de la pression proximale, via le capteur de pression proximale 7 apporte une performance supplémentaire au dispositif 1, grâce à la grande précision des mesures de pression opérées, et aussi un confort d'utilisation pour le patient, grâce à une meilleure détection de l'effort respiratoire du patient, qui permettent d'obtenir une synchronisation efficace, voire (quasi-)optimale, entre appel de gaz par le patient et fourniture de gaz par la turbine lors des phases inspiratoires, et à l'inverse, le rejet des gaz expirés par la valve expiratoire 11.

L'appareil 1 comprend aussi des moyens de réglage ou de sélection, telles des touches virtuelles affichées par l'afficheur, i.e. écran digital 14, d'une interface graphique utilisateur 9 ou IGU. L'écran d'affichage 14 est de préférence de type tactile et/ou à affichage en couleurs. Il comprend des touches de sélection ou de validation, notamment des touches tactiles affichées sur l'écran.

La turbine 2, les moyens de pilotage 4, le circuit interne 3, le capteur de débit 5 et le dispositif anti-retour 6 sont agencés dans la coque ou carcasse 1.1 périphérique de l'appareil 1, par exemple une carcasse en polymère rigide ou un autre matériau adapté.

D'une façon générale, sont prévus aussi des moyens d'alimentation électrique, telle une liaison électrique secteur (110/220V), permettent d'alimenter électriquement l'appareil 1, en particulier tous les composants nécessitant de l'énergie électrique pour fonctionner, tels les moyens de pilotage 4, l'IGU 9, l'écran 14, les capteurs 5, 7... De préférence, comme illustré en Fig. 2, les moyens d'alimentation électrique peuvent comprendre un ensemble 12 comprenant un câble électrique avec transformateur de courant et prise de liaison au secteur venant se raccorder à un connecteur électrique 13 de l'appareil 1.

En outre, l'appareil 1 peut comprendre aussi des moyens de mise en route et/ou d'arrêt, comme une touche d'allumage/d'extinction (i.e. touche « On/Off » en anglais) ou équivalent.

Durant le fonctionnement de l'appareil 1, lors des phases inspiratoires du patient, la turbine 2 est pilotée pour délivrer un débit (Q_{réglé}) décroissant ou constant de gaz, de préférence constant ou approximativement constant, selon le (les) réglage(s) ou paramètre(s) de contrôle entré(s), fixé(s) ou sélectionné(s) par l'utilisateur via l'IGU 9, en particulier une valeur de pression maximale, aussi appelée seuil de pression maximale (Pₘₐₓ) et éventuellement un temps maximum d'inspiration (Ti), c'est-à-dire une durée maximale pendant laquelle du gaz est délivré au patient pendant chaque phase inspiratoire incluant les durées Dp et Di ci-dessous.

Selon d'autres modes de réalisation, on peut aussi sélectionner
d'autres paramètres, tel que débit d'inspiration, pente inspiratoire et/ou expiratoire, sensibilité de déclenchement, temps de thérapie, etc...

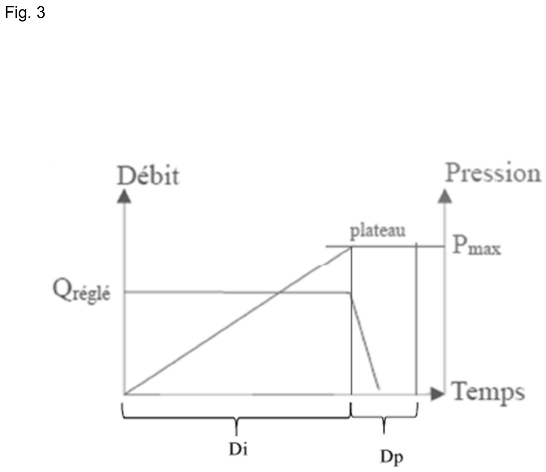

Plus précisément, comme illustré en Fig. 3, selon l'invention, les moyens de pilotage 4, typiquement un microprocesseur, sont configurés pour commander la turbine 2, typiquement le moteur électrique de la turbine, lors de chaque phase inspiratoire du patient, pour d'abord fournir un débit gazeux (Q_{réglé}) contrôlé/désiré, typiquement un débit d'air, de préférence constant, pendant une durée d'insufflation de gaz (Di) non-nulle suffisante pour atteindre un seuil de pression maximale (Pₘₐₓ) préfixé, par exemple compris entre 10 et 50 cmH2O.

Ensuite, lorsque la pression instantanée mesurée (Pₘₑₛ) par le capteur de pression proximale 7 atteint le seuil de pression maximale (Pₘₐₓ) préfixé, i.e. Pₘₑₛ = Pₘₐₓ, les moyens de pilotage 4 cessent de piloter la turbine 2 en débit et commencent à la piloter en pression. A ce moment, la turbine 2 qui n'est plus pilotée en débit et elle ralentit, ce qui engendre une chute, préférentiellement progressive, du débit de gaz fourni.

Toutefois, comme illustré en Fig. 3, selon l'invention, on opère alors un maintien d'au moins une partie du circuit patient 10 externe à une pression de plateau (Pₚₗₐ) égale au seuil de pression maximale (Pₘₐₓ) préfixé, i.e. Pₚₗₐ = Pₘₐₓ, pendant une durée de plateau (Dp) non-nulle donnée, par exemple une durée de plateau (Dp) comprise entre 0,5 et 5 sec. En général, la durée d'insufflation de gaz (Di) est supérieure à la durée de plateau (Dp).

Pour ce faire, les moyens de pilotage 4 commandent la turbine 2 pour fournir une pression de plateau (Pₚₗₐₜ) correspondant au seuil de pression maximale (P_{max.} )

Autrement dit, pendant chaque phase inspiratoire du patient, selon l'invention, les moyens de pilotage 4 commandent d'abord la turbine 2 en débit puis, lorsque la pression de plateau est atteinte, à l'issue de la durée d'insufflation de gaz Di, ils la commandent en pression pendant la durée de plateau Dp.

Durant toute la phase inspiratoire du patient, la valve expiratoire 11 reste fermée, c'est-à-dire pendant les durées Di et Dp.

Fig. 3 illustre des courbes de débit (Q) et de pression (P) en fonction du temps, pendant une phase inspiratoire du patient, le débit réglé Q_{réglé} par l'utilisateur et délivré par la turbine 2 au patient est maintenu constant pendant la durée d'insufflation de gaz Di, par exemple, un débit réglé Q_{réglé} compris entre 5 et 100 L/min.

La durée d'insufflation de gaz Di correspond au temps nécessaire pour augmenter progressivement la pression gazeuse dans le circuit patient 10 externe, donc aussi dans les poumons du patient, jusqu'à atteindre une pression maximale souhaitée, à savoir le seuil de pression maximale Pₘₐₓ préfixé. Ici, l'augmentation de la pression gazeuse est linéaire ; toutefois, elle pourrait être non-linéaire étant donné que l'augmentation de la pression est la résultante du débit gazeux fourni aux poumons du patient, c'est-à-dire du volume de gaz qui y pénètre. Cette augmentation de la pression gazeuse peut être (très) variable en fonction notamment de la compliance et/ou de la résistance des poumons du patient.

On voit qu'une fois le seuil de pression maximale Pₘₐₓ atteint, la pression de gaz dans le circuit patient 10 est maintenue à la valeur du seuil de pression maximale Pₘₐₓ de manière à obtenir un plateau de pression (Plateau), c'est-à-dire une pression de plateau Pₚₗₐ égale à Pₘₐₓ régnant dans le circuit patient 10, en particulier dans sa partie aval à proximité du patient et de ses voies aériennes.

Cette pression de plateau Pₚₗₐ est maintenue pendant la durée de plateau Dp, alors que, dans le même intervalle de temps, le débit diminue progressivement de sa valeur réglée Q_{réglé} à une valeur inférieure, par exemple une valeur nulle ou proche de la valeur nulle (i.e. 0 L/min). Cette diminution progressive du débit correspond au ralentissement ou freinage de la turbine 2 qui est alors pilotée en pression (et non plus en débit) pour maintenir la pression de plateau Pₚₗₐ.

Grâce à l'instauration de ce plateau de pression (Plateau), il s'opère un maintien du circuit patient externe 10 à la pression de plateau Pₚₗₐ qui est égale au seuil de pression maximale Pₘₐₓ préfixé, pendant la durée de plateau Dp, ce qui conduit à augmenter le confort pour le patient et l'efficacité de son traitement global, étant donné que la phase de plateau de pression permet d'augmenter le volume de gaz, i.e. d'air, délivré au patient et d'améliorer ainsi la propagation du gaz dans ses poumons et ce, sans pour autant augmenter la pression maximale de coupure, c'est-à-dire la pression maximale délivrée, donc en limitant les éventuels effets secondaires du traitement de type barotrauma.

Comme déjà dit, grâce à la phase de plateau de pression, un personnel soignant, i.e. un clinicien ou analogue, peut adapter la thérapie du patient pour obtenir le meilleur compromis possible entre la pression maximale et l'efficacité du traitement.

Selon l'invention, les moyens de pilotage 4 opèrent donc une comparaison entre les mesures de pression proximale Pₘₑₛ provenant du capteur de pression proximale 7 et le seuil de pression maximale Pₘₐₓ préfixé, et déclenchent la phase de plateau et un maintien à la pression de plateau Pₚₗₐ, lorsque ces pressions Pₘₑₛ et Pₘₐₓ deviennent égales l'une à l'autre.

A la fin de la durée de plateau Dp, on stoppe le pilotage en pression de la turbine 2 et on assiste à une chute brutale de la pression régnant dans le circuit interne 3 de l'appareil 1, en particulier au niveau du piquage 8.1 de la ligne de pilotage de la valve expiratoire 11, donc au sein de la ligne de pilotage de la valve expiratoire 11 elle-même. Cette baisse brutale de pression va alors engendrer une ouverture de la valve expiratoire 11 afin d'évacuer au moins une partie de la pression gazeuse présente dans le circuit patient 10 vers l'atmosphère extérieure et d'obtenir ainsi une baisse de pression dans le circuit patient 10, comme détaillé ci-après.

Toutefois, on note par ailleurs qu'un débit gazeux résiduel est maintenu pendant au moins une partie de la durée de plateau Dp. Ici, le débit résiduel décroit progressivement selon un profil linéaire ou non-linéaire, par exemple sensiblement parabolique ou autre.

Les paramètre(s) de contrôle, en particulier le seuil de pression maximale (Pₘₐₓ), la durée de plateau (Dp) et/ou le débit réglé Q_{réglé}, peuvent être mémorisés au sein de moyens de mémorisation de l'appareil 1, telle une mémoire flash, RAM, EEPROM ou analogue.

Ces paramètres peuvent être réglés via des moyens de réglages de pression, de débit et/ou de durée, telles une ou des touches ou boutons de réglage, par exemple des touches virtuelles ou analogues affichées par l'IGU.

D'une façon générale, comme illustré en Fig. 1, le gaz provenant de la turbine 2, pendant son fonctionnement, est délivré au patient après avoir été convoyé par le circuit de gaz 3 interne, donc via le capteur de débit 5 et le dispositif anti-retour 6 (i.e. clapet anti-retour ou analogue).

La ligne de pilotage pneumatique 8, i.e. tuyau flexible ou analogue, servant au pilotage de la valve expiratoire 11 est connectée au circuit de gaz 3 interne, en aval (en 8.1) de la sortie de la turbine 2, ce qui permet une fermeture de la valve expiratoire 11, typiquement par action sur les moyens d'étanchéité flexibles de la valve expiratoire 11, pendant l'inspiration du patient, c'est-à-dire pendant les phases d'inspiration et de plateau (i.e. pendant les durées Di et Dp), grâce à la pression gazeuse provenant de la turbine 2 qui se propage via la ligne de pilotage pneumatique 8 jusqu'à la valve expiratoire 11.

Préférentiellement, les moyens d'étanchéité flexibles de la valve expiratoire 11 comprennent un ballonnet ou une membrane flexible/déformable ou analogue, et un orifice de mise à l'atmosphère ou évent en liaison avec l'atmosphère, pour contrôler le flux gazeux, c'est-à-dire pour bloquer toute sortie de gaz via ledit orifice de mise à l'atmosphère en empêchant ainsi toute sortie de gaz pendant les phases inspiratoires, ou à l'inverse libérer plus ou moins cet orifice pour autoriser alors le passage de gaz vers l'atmosphère, donc sa sortie du circuit patient 10 externe. Une telle architecture est classique.

Autrement dit, les moyens d'étanchéité flexibles sont agencés dans le corps de valve et coopèrent avec une partie de la paroi dudit corps de valve à la manière d'un clapet et d'un siège de clapet pour contrôler les flux gazeux au travers du corps de valve et leur libération à l'atmosphère.

Dans un mode de réalisation, la valve expiratoire 11, i.e. valve d'expiration, peut comprendre en outre une zone étanche comprise entre la membrane et le corps supérieur de la vanne 11 qui permet le pilotage en fonction de la pression qui est envoyée dans la zone étanche par le biais du piquage de pilotage.

Par ailleurs, la (les) mesure de pression proximale opérée(s) par le capteur de pression proximale 7 reflète(nt) la pression s'exerçant dans le circuit patient 10 au plus près du patient (en 7.2), c'est-à-dire au voisinage immédiat de ses voies respiratoires.

Quand la pression proximale mesurée au plus près du patient atteint la valeur de pression maximale (pré)réglée, c'est-à-dire le seuil de pression maximale (Pₘₐₓ) préfixé, voire quand un temps maximum d'inspiration (pré)fixé ou réglé est atteint, les moyens de pilotage 4 contrôlent la turbine 2, plus précisément son moteur électrique, de sorte que celle-ci ralentisse et/ou freine, c'est-à-dire que les rotations du moteur 2 diminuent et/ou stoppent, mais tout en maintenant une pression positive dans le circuit 10 à la valeur de pression de plateau Pₚₗₐ.

Comme déjà dit, les comparaisons entre les valeurs de pression proximale mesurée et de pression maximale préfixée, c'est-à-dire le seuil de pression maximale (Pₘₐₓ) préfixé, sont opérées par les moyens de pilotage 4, typiquement par un microprocesseur.

Par ailleurs, les moyens de pilotage 4 peuvent en outre comprendre un compteur temporel ou analogue pour déterminer les durées ou autres périodes de temps.

Du fait de la présence du dispositif anti-retour 6, toute diminution ou chute de pression n'est pas directement transmise au circuit patient 10 mais reste dans le circuit interne 3 et dans la ligne de pilotage 8 de la valve d'expiration 11, ce qui permet à la valve 11 de s'ouvrir, après la phase de plateau, puisque la pression s'exerçant dans le ballonnet ou sur la membrane de la valve 11 diminue, et dès lors de laisser le gaz s'échapper à l'atmosphère, donc le patient expirer au travers de ladite valve expiratoire 11 qui communique alors fluidiquement avec l'atmosphère, via son orifice de mise à l'atmosphère étant donné que le ballonnet ou la membrane n'assure plus l'étanchéité gazeuse.

L'expiration du patient se déroule alors de façon passive à travers la valve d'expiration 11 du circuit patient 10 et vers l'atmosphère, i.e. au travers du corps de valve et de l'orifice de mise à l'atmosphère.

Plus généralement, dans l'appareil de l'invention, l'utilisation de la pression proximale pour la synchronisation des phases inspiratoires et expiratoires du patient permet une meilleure sélectivité du système qui n'est plus influencée par le débit venant de la turbine 2 et par les résistances pneumatiques du circuit patient 10.

La détection de l'effort respiratoire du patient par l'appareil 1 se fait de manière plus précise et plus sensible, ce qui permet d'améliorer le traitement IPPB, notamment du fait d'une meilleure synchronisation de la délivrance de gaz à l'effort respiratoire du patient.

Lorsque l'on examine Fig. 3, on constate que, pendant l'inspiration, la turbine 2 délivre d'abord le débit gazeux désiré Q_{réglé} qui peut être décélérant ou constant, à savoir ici constant, en fonction des réglages opérés par l'utilisateur via l'interface ou IGU.

Le débit Q_{réglé} est délivré au patient à travers du capteur de débit 5 et du clapet anti-retour 6. Le débit Q_{réglé} est régulé en boucle fermé par les moyens de pilotage 4, tel un calculateur, à partir de mesures de débit opérées en sortie/en aval de la turbine 2. La vitesse de la turbine 2 peut ainsi être adaptée par les moyens de pilotage 4 en fonction du profil de débit désiré. La régulation, par exemple de type proportionnelle intégrale dérivée (PID), permet de maintenir une différence minimale entre le profil de débit souhaité et le profil de débit résultant de l'adaptation de la vitesse turbine et ce quelle que soit la pression résultante, à condition qu'elle demeure en dessous du seuil de pression maximale Pₘₐₓ de coupure de l'inspiration.

Grâce au plateau de pression (plateau), on ajoute une phase de plateau à la fin de l'inspiration avec maintien de pression constante, i.e. une pression de plateau Pₚₗₐ égale à Pₘₐₓ. Autrement dit, le ventilateur 1 opère alors une régulation en pression avec maintien de la pression maximale Pₘₐₓ, pendant le temps réglé par l'opérateur, à savoir la durée de plateau (Dp) fixée par l'utilisateur.

Ensuite, au moment du passage à la régulation de pression, i.e. au début de la phase de plateau, la composante de pression liée aux résistances du patient doit être préférentiellement rapidement compensée par le système de régulation pour éviter une chute de la pression et un inconfort pour le patient.

Dans le cas de l'utilisation d'une régulation PID, le passage d'une régulation de débit à une régulation de pression nécessite une réinitialisation de certains paramètres, tels que le facteur intégral, pour éviter la chute de la vitesse turbine pendant la phase de chargement du facteur intégral.

Idéalement, la pression est parfaitement maintenue à la pression maximale pendant cette phase et le patient ne ressent aucune transition d'une phase à l'autre.

D'une façon générale, l'appareil de thérapie respiratoire selon l'invention est bien adapté à la fourniture d'une thérapie respiratoire de type IPPB aux patients en ayant besoin, typiquement aux personnes souffrant de problèmes ou pathologies respiratoires nécessitant d'améliorer leur drainage bronchique ; leur fonction respiratoire ; et leur recrutement pulmonaire, en particulier des zones pulmonaires mal ou non ventilées.

## Revendications

1. Appareil de thérapie respiratoire (1) pour fournir une assistance respiratoire intermittente par pression positive (IPPB) à une personne, comprenant :
- une coque ou carcasse périphérique (1.1),
- une turbine (2) motorisée pour fournir un gaz respiratoire sous pression,
- un circuit de gaz (3) interne pour convoyer le gaz respiratoire délivré par la turbine (2),
- un capteur de débit (5) agencé sur le circuit de gaz (3) interne,
- un dispositif anti-retour (6) agencé dans le circuit de gaz (3) interne, en aval du capteur de débit (5),
- un circuit patient (10) externe venant se raccorder fluidiquement au circuit de gaz (3) interne pour convoyer le gaz respiratoire provenant du circuit de gaz (3) interne,
- un capteur de pression proximale (7) agencé pour permettre une mesure de la pression au sein du circuit patient (10) externe, et
- des moyens de pilotage (4) raccordés au capteur de pression proximale (7) et à la turbine (2) motorisée, et configurés pour commander la turbine (2) en fonction d'au moins une mesure de pression opérée par le capteur de pression proximale (7) et d'au moins une mesure de débit opérée par le capteur de débit (5),
et dans lequel la turbine (2), les moyens de pilotage (4), le circuit interne (3), le capteur de débit (5) et le dispositif anti-retour (6) sont agencés dans la coque ou carcasse périphérique (1.1),
**caractérisé en ce que** les moyens de pilotage (4) sont configurés pour, lors de chaque phase inspiratoire de la personne :
a) commander la turbine pour fournir un débit gazeux régulé (Q_{réglé}), pendant une durée d'insufflation de gaz (Di) non-nulle suffisante pour atteindre un seuil de pression maximale (Pₘₐₓ) fixé, et
b) lorsqu'une pression mesurée (Pₘₑₛ) par le capteur de pression proximale est supérieure ou égale au seuil de pression maximale (Pₘₐₓ) préfixé, commander la turbine pour fournir une pression de plateau (Pₚₗₐₜ) correspondant au seuil de pression maximale (Pₘₐₓ) de manière à maintenir au moins une partie du circuit patient externe à ladite pression de plateau (Pₚₗₐₜ), pendant une durée de plateau (Dp) non-nulle donnée.

2. Appareil selon la revendication 1, **caractérisé en ce qu'**il comprend en outre :
- une valve expiratoire (11) agencée sur le circuit patient (10) externe, et
- une ligne de pilotage pneumatique (8) de la valve expiratoire (11) venant se raccorder fluidiquement (8.1) au circuit de gaz (3) interne entre la turbine (2) et le capteur de débit (5), et coopérant avec la valve expiratoire (11) pour contrôler l'ouverture ou la fermeture de ladite valve expiratoire (11).

3. Appareil selon la revendication 2, **caractérisé en ce que** le capteur de pression proximale (7) est agencé pour permettre une mesure de la pression au sein du circuit patient (10) externe via une ligne de mesure de pression (7.1) venant se raccorder au circuit patient (10) en aval (7.2) de la valve expiratoire (11).

4. Appareil selon la revendication 1, **caractérisé en ce que** les moyens de pilotage (4) sont configurés pour commander la turbine (2) pour que le débit gazeux (Q_{réglé}) fourni à l'étape a), soit un débit constant ou décélérant pendant la durée d'insufflation de gaz (Di).

5. Appareil selon la revendication 1, **caractérisé en ce qu'**il comprend des moyens de réglage de durée configurés pour permettre à l'utilisateur de régler la durée de plateau (Dp) et/ou des moyens de réglage de pression configurés pour permettre à l'utilisateur de régler le seuil de pression maximale (Pₘₐₓ).

6. Appareil selon les revendications 1 et 2, **caractérisé en ce que** le capteur de pression proximale (7) est configuré pour permettre une mesure de la pression au sein du circuit patient (10) externe entre (7.2) la valve expiratoire (11) et une interface respiratoire (20) assurant les échanges gazeux avec le patient.

7. Appareil selon la revendication 1, **caractérisé en ce que** lorsque lesdits moyens de pilotage (4) déterminent que la pression proximale mesurée (Pₘₑₛ) atteint le seuil de pression maximale (Pₘₐₓ), les moyens de pilotage (4) sont configurés pour cesser de piloter la turbine (2) en débit et commencer à la piloter en pression de manière à ralentir la turbine (2) et engendrer une chute du débit de gaz fourni.

8. Appareil selon la revendication 1, **caractérisé en ce que** les moyens de pilotage (4) sont configurés pour comparer une valeur de pression proximale mesurée (Pₘₑₛ) par le capteur de pression proximale (7) au seuil de pression maximale (Pₘₐₓ) fixé et déclencher l'étape b) lorsque la valeur de pression mesurée (Pₘₑₛ) par le capteur de pression proximale (7) est égale au seuil de pression maximale (Pₘₐₓ) préfixé.

9. Appareil selon la revendication 2, **caractérisé en ce que**, pendant la durée de plateau (Dp), la valve expiratoire (11) est fermée de manière à maintenir d'au moins une partie du circuit patient (10) externe à la pression de plateau (Pₚₗₐ).

10. Appareil selon la revendication 2, **caractérisé en ce qu'**à la fin de la durée de plateau (Dp), la valve expiratoire (11) s'ouvre de manière à évacuer au moins une partie de la pression gazeuse présente dans le circuit patient (10) externe.

11. Appareil selon la revendication 1, **caractérisé en ce que** le seuil de pression maximale (Pₘₐₓ) est compris entre 10 et 50 cmH2O.

12. Appareil selon la revendication 1, **caractérisé en ce que** les moyens de pilotage (4) comprennent un compteur temporel.

13. Appareil selon la revendication 1, **caractérisé en ce que** les moyens de pilotage (4) comprennent au moins un microprocesseur.

14. Appareil selon la revendication 1, **caractérisé en ce que** la turbine (2) motorisée comprend un moteur électrique.

15. Appareil selon la revendication 14, **caractérisé en ce que** les moyens de pilotage (4) sont configurés pour commander les accélérations et les décélérations ou freinages dudit moteur électrique de la turbine (2).
